# EUROPÄISCHE PATENTSCHRIFT

(11) **EP 1 348 123 B1**
(45) Veröffentlichungstag und Bekanntmachung des Hinweises auf die Patenterteilung: **16.03.2005**
(21) Anmeldenummer: 01909430.9
(22) Anmeldetag: 05.01.2001
(51) Int. Cl.: G01N 27/62, H01J 49/04, G01N 1/42, G01N 1/00

(54) **ANALYSEVERFAHREN ZUR DETEKTION VON RÄUMLICHEN SPURENELEMENT-VERTEILUNGSMUSTERN UND VORRICHTUNG ZUR VERFAHRENSDURCHFÜHRUNG**
ANALYSIS METHOD FOR DETECTING THREE-DIMENSIONAL TRACE ELEMENT DISTRIBUTION PATTERNS AND CORRESPONDING DEVICE FOR CARRYING OUT THIS METHOD
PROCEDE D'ANALYSE PERMETTANT LA DETECTION DE MOTIFS DE REPARTITION SPATIALE D'OLIGO-ELEMENTS, ET DISPOSITIF SERVANT A METTRE EN OEUVRE LE PROCEDE

(43) Veröffentlichungstag der Anmeldung: 01.10.2003
(73) Patentinhaber: Stiftung Alfred-Wegener-Institut für Polar- und Meeresforschung, 27568 Bremerhaven (DE); GESELLSCHAFT ZUR FÖRDERUNG DER SPEKTROCHEMIE UND ANGEWANDTEN SPEKTROSKOPIE E.V., 44139 Dortmund (DE); Impres GmbH Ingenieurbüro, 282845 Bremen (DE)
(72) Erfinder: KRIEWS, Michael, 27580 Bremerhaven (DE); DUNKER, Erich, 21762 Otterndorf (DE); REINHARDT, Heiko, 25335 Elmshorn (DE); BENINGA, Ingo, 28259 Bremen (DE); HOFFMANN, Erwin, 16540 Neuendorf (DE); LÜDKE, Christian, 12555 Berlin (DE)
(86) Internationale Anmeldenummer: PCT/DE2001/000028
(87) Internationale Veröffentlichungsnummer: WO 2002/054057

(56) Entgegenhaltungen:
- WO-A-95/09688
- DE-A- 19 529 717
- DE-A- 19 934 561
- US-A- 4 920 264
- PATENT ABSTRACTS OF JAPAN vol. 1999, no. 05, 31. Mai 1999 (1999-05-31) & JP 11 051904 A (JEOL LTD), 26. Februar 1999 (1999-02-26)

## Beschreibung

Die Erfindung bezieht sich auf ein Analyseverfahren zur Detektion von räumlichen Spurenelement-Verteilungsmustern in einer Feststoffprobe mittels rechnergestütztem, ortsaufgelöstem Abtragen von partikulärem Probenmaterial durch den Beschuss mit Laserstrahlung in einer von einem inerten Trägergasstrom durchströmten Probenkammer und anschließender Überführung des herausgelösten Probenmaterials mit Hilfe des Trägergasstromes in ein induktiv gekoppeltes Plasma als Ionisierungsquelle zur Messdatenaufnahme durch ein Massenspektrometer und auf eine Vorrichtung zur Verfahrensdurchführung.

Eisschichten und Gletscher in polaren Regionen entstehen durch kontinuierliche Deposition von Schnee. Aerosole marinen, terrestrischen, anthropogenen und kosmischen Ursprungs lagern sich auf dem polaren Schnee und Eis ab. So dienen die riesigen Schnee- und Eisflächen der Polargebiete als Klimaarchive und liefern einen wertvollen Einblick in bis zu 250.000 Jahre Erdklimageschichte. Umweltbedingte Veränderungen können als chemische und physikalische Parameter in Eiskemen detektiert werden, die aus den Eisschichten und Gletschern herausgebohrt werden. Unter den chemischen Parametern interessieren insbesondere die Spurenelemente, da durch sie auf unbeeinflusste Umweltveränderungen und Umweltverschmutzungen durch anthropogene Einwirkung geschlossen werden kann. Analytische Techniken wurden entwickelt, um derartige Ultraspuren in den zur Verfügung stehenden, äußerst begrenzten Eiskernvolumina nachweisen zu können. Dabei stehen Faktoren wie eine hohe zeitliche und örtliche Auflösung und eine möglichst geringe Kontamination des Probenmaterials während der Analyse im Vordergrund.

In dem Aufsatz "Determination of the Trace Elements in a Mizuho Ice Core Sample by a Combination of Conventional and High Resolution Inductively Coupled Plasma Mass Spectrometry" von T. Shimamura et al. (Proc. NIPR Symp. Polar Meteorol. Glaciol., 9, 33-44, 1995) wird ein Verfahren beschrieben, mit dem die Detektion von 17 verschiedenen Spurenelementen in einem Eiskern aus der Antarktis möglich war. Diese Verfahren basiert auf einer atommassenspektrometischen Analyse des zu untersuchenden Materials mit einem induktiv gekoppelten Plasma als Ionenquelle (ICP-MS - Inductively Coupled Plasma - Mass Spectrometry). Die Analyse der in dem Plasma ionisierten Materialprobe kann über ein Quadrupol-Massenspektrometer (Q-ICP-MS - Quadru-pole based) oder über ein Massenspektrometer mit einer höheren Auflösung (HR-ICP-MS - High Resolution) erfolgen. In Abhängigkeit von der Auflösung können verschiedene Spurenelemente in unterschiedlichen Vorkommen detektiert werden. Während der Analyse erreicht das ICP eine Temperatur zwischen 8.000 K und 10.000 K und wird durch einen Gasstrom gekühlt. Eingebracht in das heiße Plasma wird die präparierte Probe über einen speziellen Zerstäuber. Für ein derartiges Verfahren muss die Eisprobe von außen nach innen stufenweise aufgeschmolzen werden, wodurch die Ortsauflösung stark verringert wird. Weiterhin sind viele Störfaktoren zu berücksichtigen, insbesondere die Kontamination der Probe während der Probenaufbereitung durch Kontakt mit Lösungsmitteln und wechselnden Gefäßen und der Analyse durch die Messeinrichtung selbst.

Aus dem Aufsatz "Determination of trace Elements in an Arctic Ice Core by ICP/MS with a Desolvated Micro-concentric Nebulizer" von S. Matoba et. al. (Journal of Environmental Chemistry Vol. 8, No.3, pp.421-427, 1998) ist eine sehr umfangreiche Probenpräparation bekannt, die der Minimierung des Kontaminationsproblems dienen soll. Dabei wird eine oberflächlich abgeschabte Eisprobe zunächst stufenweise zur Vermeidung von Rissen auf Zimmertemperatur in einem gekühlten Reinraum erwärmt, dann mit hochreinem Wasser in einem Reinraum gewaschen und danach in verschiedenen Teflon-Behältern zur Vermeidung von Kontamination 50g-weise aufgeschmolzen und der verbleibende Probenrest wieder eingefroren. Zur Fertigstellung der einzelnen Proben, beispielsweise fünf an der Zahl, wird der aufgeschmolzenen Probe jeweils noch Salpetersäure zugefügt. Bei dem hier verwendeten Zerstäuber handelt es sich um einen Mikro-Konzentrischen Zerstäuber (MCN), der Vorteile gegenüber den bekannten pneumatischen Zerstäubern und Ultraschall-Zerstäubern aufweist.

Bei den genannten Verfahren mit den beschriebenen chemisch-physikalischen Methoden zur elementanalytischen Untersuchung von Eisbohrkernen ist es Voraussetzung, dass die Proben in aufwändigen Präparationsverfahren zunächst aufgeschmolzen, dann angereichert und schließlich mit Chemikalien versetzt werden müssen, um sie über den Zerstäuber in die Plasmaflamme einsprühen zu können. Durch die relativ großen Volumina in der Lösungsanalytik verringert sich die Ortsauflösung in starkem Maße und damit auch die Zeitauflösung (Jahresschichten) der detektierten Spurenelemente innerhalb einer Eisprobe. Neben der zeitaufwändigen Präparation ist insbesondere das hohe Kontaminationsrisiko bei den präparierten Eisproben durch deren Kontakt mit unterschiedlichen Flüssigkeiten, Gefäßen und Verfahrensvorrichtungen ein großer Nachteil der bekannten Verfahren, da eine auftretende Probenverschmutzung zu einer starken Verfälschung der Messergebnisse führt.

Aus der US-PS 4.920.264 ist ein Verfahren zur Präparierung bzw. Aufbereitung großer organischer Moleküle bekannt, um diese einer Massenspektrometrie zugänglich zu machen. Hierzu werden schwerflüchtige oder thermisch labile hochmolekulare Verbindungen hergestellt und in einer Matrix aus einem niedermolekularen Lösungsmittel eingefroren. Dadurch werden unerwünschte Fragmentierungen oder Clusterbildungen bei der Desorption vermieden. Der Gefrierzustand der eingefrorenen Lösung wird in Abhängigkeit des Dampfdruckes in einer Vakuumkammer aufrecht erhalten. Nach der hier erfolgenden Desorption werden die hochmolekularen organischen Substanzen photoionisiert oder durch Laserstrahlbeschuss ionisiert und einem Massenspektrometer für organische Analytik zugeführt. Dabei muss bei der Wahl sowohl der Desorptions- als auch der Ionisierungsenergie darauf geachtet werden, dass die empfindlichen Moleküle nicht zerstört werden. Das beschriebene Präparations- bzw. Aufbereitungsverfahren mit dem Ausgangsmaterial einer homogenen Lösung ist daher nicht anwendbar für eine anschließende Detektion von anorganischen Spurenelementen, die erst bei sehr hohen Temperaturen ionisieren, da die eingesetzte Laserenergie nicht ausreichend ist. Eine ortsabhängige Detektion von Spurenelementen zur Ermittlung des qualitativen und quantitativen Verteilungsmusters kann mit dem bekannten Verfahren gemäß der US-PS 4.920.264 nicht durchgeführt werden.

Das Analyseverfahren, von dem die Erfindung als nächstliegendem **Stand der Technik** ausgeht (beschrieben beispielsweise in dem Handbuch (Ausgabe 1991) zu dem "Laser-Zubehör Modell 320" der Firma Perkin-Elmer, hier insbesondere die Seiten 1-1 bis 1-6), basiert auf einer direkten Verdampfung von geringsten Probenmengen aus der Festkörperphase durch örtlich hochauflösbare Laserbestrahlung und Zuführung des verdampften Probenmaterials in das Plasma über ein inertes Trägergas. Somit können Feststoffproben unter Umgehung des üblichen Probenaufschlusses und der Zerstäubung der Probenflüssigkeit direkt in das induktiv gekoppelte Plasma eingebracht werden. Hierdurch lassen sich eine Reihe typischer analytischer Probleme vermeiden, wie sie beim Einsatz von konventionellen Techniken mit Probenaufschluss und anschließender Vernebelung auftreten. Durch den Laserstrahl verdampfte Probenpartikel werden mittels eines Überführungssystems mit sehr hoher Effizienz in das Plasma übertragen. Mit dem beschriebenen Verfahren der Laserverdampfung kann beinahe jede Feststoffprobe analysiert werden. Die Proben können als Stäbe, Scheiben, Blöcke, Draht, Pulver oder Späne vorliegen. Insbesondere wurden bislang mit Erfolg beispielsweise Reinstmetalle, Oxide, supraleitfähige und geologische Materialien, Glas, Keramikwerkstoffe, Halbleitermaterialien, Polyethylen und Teflon, analysiert. Auch sogenannte "Schmelzen", bei denen es sich um wiedererstarrte, durch vorheriges Aufschmelzen homogenisierte Feststoffe handelt, wurden erfolgreich untersucht. Allen diesen Materialien, die sich für eine Untersuchung mit Laserverdampfung eignen, ist jedoch gemeinsam, dass sie bei Raumtemperatur in einem festen Aggregatzustand vorliegen.

Eisproben, deren große Bedeutung eingangs beschrieben wurde, können demgemäß mit dem bekannten Analyseverfahren mit Laserverdampfung bislang nicht analysiert werden, weil sie bei Raumtemperatur nur in flüssiger Form vorliegen. Außerdem ergeben sich bei einer geschmolzenen Eisprobe die bereits weiter oben ausführlich behandelten Nachteile, insbesondere die der Kontamination und der geringen Orts- und Zeitauflösung, sodass jedes Aufschmelzen dringend vermieden werden sollte. Das o.g. bekannte Präparationsverfahren hingegen mit einer laserstrahlunterstützten Ionisierung einer zuvor eingefrorenen homogenisierten Lösung stellt nur die Vorbereitungsstufe für ein Analyseverfahren dar. Eine ortsaufgelöste Detektion von Spurenelementen in einer Feststoffprobe ist mit diesem Verfahren nicht möglich.

Es ist daher **Aufgabe** der Erfindung, ein Analyseverfahren zur Detektion von räumlichen Spurenelement-Verteilungsmustern in einer Feststoffprobe mit einer direkten Verdampfung durch örtlich hochauflösbare Laserbestrahlung der oben genannten Art so zu modifizieren, dass auch Materialien, die bei Raumtemperatur in einem flüssigen Aggregatzustand vorliegen, aber einen Gefrierpunkt unterhalb der Raumtemperatur aufweisen, untersucht werden können. Dabei sind einfache Maßnahmen zu ergreifen, die das Verfahren weder apparate- noch zeitaufwändiger und noch kostenintensiver machen. Eine einfache Handhabung durch das Personal, auch bei einer entsprechenden Vorrichtung, ist zu gewährleisten.

Die Lösung dieser Aufgabe wird bei dem erfindungsgemäßen Analyseverfahren dadurch bewirkt, dass die Feststoffprobe in Form einer natürlichen Eisprobe oder einer gefrorenen biologischen Probe mit charakteristisch festem Ausgangszustand in der Probenkammer angeordnet ist und zur Aufrechterhaltung des festen Ausgangszustandes der Feststoffprobe während der Verfahrensdurchführung das Innere der Probenkammer und der die Probenkammer durchströmende Trägergasstrom auf Temperaturen unterhalb des Gefrier- oder Erstarrungspunktes der Feststoffprobe gekühlt wird.

Im Zusammenhang mit gefrorenen Eisproben als Feststoffproben kann bei dem erfindungsgemäßen Verfahren nunmehr im erweiterten Sinne von einer "Laserablations-ICP-MS" gesprochen werden. Dabei bezeichnet der Begriff "Ablation" insbesondere das Abtragen von partikulärem Probenmaterial durch den Beschuss mit Laserstrahlung. Ein vollständiges Schmelzen wie bei den bekannten Analyseverfahren findet jedoch nicht mehr statt. Die Proben müssen nicht mehr aufgetaut werden und kommen dadurch mit weniger Materialien und Chemikalien in Berührung. Dadurch ergibt sich eine Reduzierung der Gefahr von Kontamination wie sie bei Aufschlusstechniken immer gegeben ist. Spektrale Störungen, wie sie nach Aufschluss häufig bedingt durch die Aufschlussreagenzien sowie das Lösungsmittel auftreten, werden weitgehend vermieden. Eine aufwändige Probenpräparation entfällt. Aus dem Eisblock werden partiell mit hoher örtlicher und daraus folgend mit hoher zeitlicher Auflösung der Spurenstoff-Verteilungsmuster Probenpartikel durch Laserstrahlbeschuss herausgelöst und zur Plasmaflamme weitergeleitet. Dabei bildet der Laserstrahlfokus den Parameter für die Aufllösungsgrenze. In Abhängigkeit von der Wellenlänge des Lasers und der Energie des auftreffenden Laserstrahls ist ein hohe räumliche Auflösung im Bereich von 20 µm bis 1000 µm erreichbar. Durch den Ablationsprozess findet eine Dekontamination der Probe und gleichzeitig eine in-situ-Kontrolle statt. Es besteht eine hohe Nachweisempfindlichkeit in der gefrorenen Feststoffprobe. Neben Eisbohrkemen können auch gefrorene biologische Proben, beispielsweise Gewebematerial, auf das räumliche Verteilungsmuster ihrer einzelnen Komponenten hin analysiert werden. Mikrostrukturen können einfach untersucht werden.

Mit der hohen räumlichen Auflösung können die durch hohen Druck entstandenen, extrem dünnen Jahresschichten (im mm-Bereich) in den tieferen Lagen von Eisbohrkernen, die durch saisonale Schwankungen der Elementkonzentration entstanden sind, noch erkannt und im Hinblick auf die Elementverteilung analysiert werden. Zur Analyse an gefrorenen Proben muss jedoch gewährleistet sein, dass während des Verfahrensablaufes in der Probenkammer eine Temperatur unterhalb des Gefrierpunktes der Feststoffprobe aufrechterhalten wird, um ein Aufschmelzen der Proben zu vermeiden. Dazu wird bei dem erfindungsgemäßen Verfahren einerseits die Probenkammer in ihrem Innem selbst gekühlt, andererseits wird aber auch der Trägergasstrom gekühlt. Dadurch wird ein Wärmeeintrag in die Probenkammer verhindert. Außerdem werden Kondensniederschläge aus dem feuchten Trägergas auf der Oberfläche der Probe vermieden, die zu verfälschten Messergebnissen führen können. Spritzwasser an der Optik und Schmelzvorgänge an der Probenoberfläche werden unterbunden. Alle Störungen durch anwesendes Wasser werden weitestgehend ausgeschaltet, da dieses ebenfalls gefriert.

Eine Verbesserung der Gewährleistung des Gefrierzustandes und eine noch größere Einflussnahme der Vorteile auf die Güte der Messergebnisse kann bei dem erfindungsgemäßen Verfahren nach einer Verfahrensfortführung insbesondere vorteilhaft dadurch erreicht werden, dass die Kühltemperatur in einem Temperaturbereich bis zu 30°C unterhalb des Gefrier- oder Erstarrungspunktes der Feststoffprobe liegt. Die Stabilität der gefrorenen Proben ist dadurch sicher gewährleistet, auch bei temporären Schwankungen der Kühltemperatur. Es stehen geeignete Kühlmittel für diesen Kühlbereich zur Verfügung. Hierbei kann nach einer nächsten Erfindungsausgestaltung insbesondere vorgesehen sein, dass die Kühlung durch Ethanol oder Silikonöl als Kühlfüssigkeit erfolgt. Ethanol (Spiritus) ist ein Alkohol mit einfacher Synthese, die wenig umweltbelastend ist, und wird häufig als einfaches Lösungsmittel eingesetzt. Silikonöl ist ein umweltverträgliches hochviskoses Leichtöl. Nach einer weiteren Ausgestaltung des erfindungsgemäßen Verfahrens wird der Trägergasstrom von dem Edelgas Argon gebildet. Hierbei handelt es sich um ein inertes Gas hoher Güte und Reinheit, mit dem beim Einsatz im bekannten Laserverdampfungsverfahren die besten Erfahrungen gemacht wurden. Auch eine Abkühlung des Argons in einen Temperaturbereich um -30°C und tiefer kann problemlos erfolgen, da der eigene Gefrierpunkt wie bei allen Edelgasen bei sehr tiefen Temperaturen (Ar : ca. -190°C) liegt.

Bei dem bekannten Analyseverfahren mit Laserverdampfung wird eine Wellenlänge des emittierten Laserlichts im Infrarot-Bereich von 1064 nm verwendet. Hierzu wird in einer, ebenfalls aus dem oben zitierten Handbuch (insbesondere Seiten 3-5 bis 3-10, 6-11/6-12 und Figur) bekannten Vorrichtung zur Durchführung des Verfahrens, auf die weiter unten noch eingegangen wird, im allgemeinen ein Nd:YAG-Laser eingesetzt. Dieser eignet sich - ggfs. in modifizierter Form - auch zum Einsatz bei dem erfindungsgemäßen Laserablations-Analyseverfahren optimal für die Untersuchung von gefrorenen Proben, wie beispielsweise Eis, Gewebe, Serum, kleine Tropfen oder Hagelkörner, da diese im infraroten Wellenlängenbereich einen hohen Absorptionskoeffizienten aufweisen. Dementsprechend kann in einer vorteilhaften Vorrichtung zur Durchführung des erfindungsgemäßen Analyseverfahrens der Laser der Laseranordnung als Infrarot-Laser ausgebildet sein. Bei anderen Proben ist es entsprechend einer nächsten Erfindungsausgestaltung vorteilhaft, wenn die Wellenlänge des emittierten Laserlichts im optimalen Absorptionsbereich der Feststoffprobe liegt. Dadurch wird stets eine optimale Materialverdampfung ermöglicht.

Erfahrungsgemäß werden mit dem bekannten Laserverdampfungsverfahren die besten analytischen Ergebnisse erzielt, wenn zu Messbeginn die einwandfreie Funktion des Systems mit geeigneten Referenzmaterialien kontrolliert und ggf. optimiert wird. Die Referenzmaterialien können im Labor selbst hergestellt oder käuflich erworben werden. Zur Erstellung von Eis-Standards für die Elementanalyse können kommerziell erhältliche Multielementstandards in verschiedenen Konzentrationen in Petrischalen eingefroren werden. Die Dicke dieser Standards beträgt im allgemeinen 1 cm. Es wurde festgestellt, dass bei dünneren Eisproben der Laserstrahl durch den Eisstandard hindurchgeht und auf den Probenträger trifft. Durch einen einfachen Gefrierprozess für die Eisstandards bei dem erfindungsgemäßen Laserablations-Analyseverfahren kann es jedoch bei der Herstellung zu Inhomogenitäten und Rissbildungen kommen. Gemäß einer anderen Fortführung der Erfindung ist es deshalb vorteilhaft, wenn Standardproben zur Durchführung von Referenzmessungen durch wiederholtes Aufsprühen einer fein zerstäubten Materiallösung auf einen Objektträger bei der gewählten Kühltemperatur bis zur Erreichung einer vorbestimmten Schichtdicke oder durch Schockgefrieren (ca. bei -30°C) einer ca. 1 cm hohen Materiallösung in einer Petrischale hergestellt werden. Durch derartige Maßnahmen kann die Erstellung von homogenen, rissfreien Material-Standards sicher gewährleistet werden.

Die bekannte Vorrichtung zur Durchführung des Laserverdampfungs-Analyseverfahrens weist ein Steuerungs- und Überwachungssystem mit einem Video-Monitor mit Fadenkreuz-Generator und einem netzbetriebenen Steuercomputer sowie eine Laseranordnung (Nd:YAG-Laser) mit einer Transfer- und Fokussierungsoptik auf. Über diese wird der Laserstrahl durch ein Abdeckfenster in die Probenkammer geleitet, die aus einer Probenzelle und einem Probenträgertisch besteht. Zur Anordnung der Feststoffprobe wird diese auf dem Tisch positioniert und dann die Probenzelle aufgesetzt und mittels Schnellverschlüssen druckdicht befestigt. Der Probentisch kann mittels rechnergesteuerter Schrittmotoren in allen Raumrichtungen bewegt werden.

Die Schrittmotorsteuerung mit hoher Auflösung ermöglicht die exakte Vorwahl der zu analysierenden Probenoberfläche, sowie die Programmierung eines Rasters, das dann anschließend während des Laser-Beschusses abgefahren wird. Der Laserbeschuss kann beliebig an nur einem Punkt, entlang einer vorgegebenen Linie oder rasterförmig über eine Fläche hinweg erfolgen. Die Markierung des Punktes, der Linie oder der Fläche kann sehr einfach per Mausklick erfolgen, gestützt über die Laser-Software. Die Tatsache, dass der Probentisch vollständig vom Rechner kontrolliert wird, ermöglicht sehr flexible Anwendungen des bekannten Systems. Zur Aufnahme von Elementtiefenverteilungen wird der Laser auf den interessierenden Punkt der Probe ausgerichtet, der notwendige Fokus justiert und dann die Probe kontinuierlich beschossen (Punktscan). Während des Beschusses dringt der Laserstrahl immer tiefer in die Probe ein (Krater), wodurch sich beispielsweise ändernde Elementverteilungen in einem Probenmaterial ermitteln lassen. Auf gleiche Weise können tiefe Rinnen durch einen kontinuierlichen Beschuss entlang einer Linie (Linienscan) erzeugt und das Material analysiert werden. Sollen die Analytkonzentrationen einer großen Oberfläche bestimmt werden, so kann entweder mit einem großen Fokusdurchmesser oder/und die Probe in einem vorgewählten Raster während des Beschusses unter dem Laserstrahl bewegt werden.

Der Aufbau der bekannten Probenkammer ist ausschließlich auf Feststoffmaterialien mit einer definierten Oberfläche für eine Analyse bei Raumtemperatur ausgerichtet. Durch eine Modifikation des Systems bei der Erfindung ist also zu gewährleisten, dass auch gefrorene Proben in festem Aggregatzustand mit dem erfindungsgemäßen Laserablationsverfahren analysiert werden können.

Dazu ist eine besondere Vorrichtung zur Durchführung des erfindungsgemäßen Elemtentanalyseverfahrens in einer oder mehreren der oben beschriebenen Ausführungsformen mit den oben beschriebenen Funktionselementen vorgesehen. Diese zeichnet sich insbesondere dadurch aus, dass die Probenkammer aus einem wärmeisolierenden, hochreinen Material besteht und einen abnehmbaren Deckel sowie eine im Innern positionierbare Probenschale aufweist, unter der ein wärmeleitender, hochreiner Metallblock mit einem integrierten, Anschlüsse aufweisenden Kanalsystem angeordnet ist, und dass eine über Ventile zuschaltbare Umlauf-Kühlvorrichtung mit einer Kühlflüssigkeit vorgesehen ist, die über wärmeisolierende Rohrverbindungen sowohl mit dem Kanalsystem in dem Metallblock als auch mit einer externen Kühlbox verbunden ist, die in ihrem Innern einen Wärmetauscher aufweist, der auf seiner wärmeren Seite mit der Trägergaszuleitung verbunden ist. Weiterhin kann insbesondere als wärmeisolierendes, hochreines Material für die Probenkammer und die Probenschale Teflon und als wärmeleitendes, hochreines Material für den Metallblock Kupfer ausgewählt sein. Diese Materialien erfüllen die an sie gestellten Anforderungen optimal und garantieren ein geringes Kontaminationsrisiko.

Die Probenkammer stellt nunmehr eine geschlossene Isolierbox dar, die gezielt gekühlt werden kann. Dadurch kann in ihrem Innern nahezu jede beliebige Kühltemperatur eingestellt und aufrechterhalten werden. Eingebrachte, gefrorene Proben laufen nicht Gefahr, während der Analyse aufzuschmelzen. Weiterhin wird auch der Trägergastrom gekühlt. Der Probenraum wird also sowohl durch die Kühlflüssigkeit als auch durch den Trägergasstrom gekühlt. Kondenserscheinungen und Anschmelzprozesse sind weitgehend ausgeschlossen. Die Trägergaskühlung erfolgt außerhalb der Probenkammer über einen einfachen Wärmetauscher, beispielsweise in Form einer mehrfach gewundenen Kühlschlange, die im Innern einer Kühlbox angeordnet ist, die mit der Kühlflüssigkeit angefüllt ist.

Weiterhin kann bei der erfindungsgemäßen Vorrichtung vorteilhaft als Teil der Laseranordnung ein Justagelaser vorgesehen sein, der Laserlicht im sichtbaren Wellenlängenbereich emittiert. Mit Hilfe dieses Justagelasers, hierbei kann es sich beispielsweise um einen Helium-Neon-Laser oder um eine Laserdiode handeln, ist wie bei einer optischen Zieleinrichtung über die Abbildung eines sichtbaren Laserpunktes auf der Probenoberfläche dessen Lage zu kontrollieren und hochgenau einzustellen. Somit ist eine in-situ-Kontrolle des Scanvorganges möglich. Bei der bekannten Vorrichtung ist erst eine Kontrolle nach dem Beschuss mit der nicht-sichtbaren Laserstrahlung auf dem generierten Fadenkreuzgitter auf dem Video-Monitor möglich. Um Wiederholungen im Zusammenhang mit der Ausführung der Vorrichtung zu vermeiden, wird an dieser Stelle zu weiteren Erläuterungen bezüglich der Ausführungsvorrichtung auf den speziellen Beschreibungsteil verwiesen.

**Ausbildungsformen der Erfindung** und Diagramme dazu werden zum weiteren Verständnis nachfolgend anhand der schematischen Figuren näher erläutert. Dabei zeigt :
- **Figur 1**: eine Vorrichtung für die Laserablation von Eisproben mit einer integrierten Darstellung des Verfahrensablaufes,
- **Figur 2**: ein Detektionsdiagramm für verschiedene Eisstandards,
- **Figur 3**: verschiedene Ablationsmuster,
- **Figur 4**: ein Detektionsdiagramm für einen Linienscan an einer Eisprobe,
- **Figur 5**: ein Detektionsdiagramm für einen Punktscan an einer Eisprobe,
- **Figur 6**: ein Detektionsdiagramm im Vergleich für verschiedene Einführsysteme,
- **Figur 7**: eine Probenkammer als Konstruktionszeichnung im Querschnitt,
- **Figur 8**: die Probenkammer gemäß Figur 7 in der Draufsicht und
- **Figur 9**: eine Darstellung der Umlauf-Kühlvorrichtung.

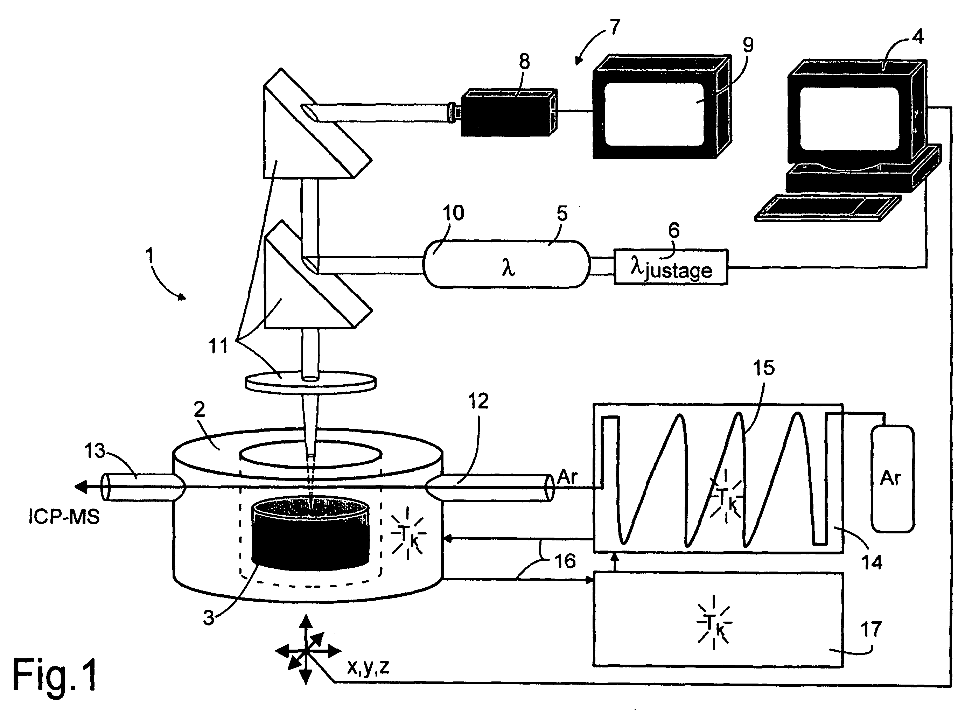

In der **Figur 1** ist schematisch eine Vorrichtung **1** zur Durchführung des erfindungsgemäßen Laserablations-ICP-MS-Verfahren zur Detektion von räumlichen Spurenelement-Verteilungsmustem in einer Feststoffprobe dargestellt. Kernstück der Vorrichtung **1** ist eine Probenkammer **2,** in der eine Feststoffprobe **3** in gefrorenem Zustand, im dargestellten Beispiel eine Eisprobe, angeordnet ist. Die Probenkammer **2** ist auf einem nicht weiter dargestellten Probentisch befestigt, der in alle drei Raumrichtungen **x,y** und **z** bewegt werden kann. Die Steuerung wird von einem Steuerungsrechner **4** übernommen, der auch der Steuerung einer Laseranordnung **5** dient, beispielsweise Modifizierung auf der Basis des Laser-Samplers 320 der Firma Perkin-Elmer/Sciex. Mit Hilfe eines Justagelasers **6** als Teil dieser Anordnung und eines Überwachungssystems **7** zum Schutz des Bedienpersonals gegenüber dem energiereichen Laserstrahl, bestehend aus einer Farb-Kamera **8** und einem Video-Monitor 9, wird die Eisprobe 3 exakt justiert. Mit einem ebenfalls vom Steuerrechner **4** gesteuerten Detektionslaser **10,** im dargestellten Beispiel ein leistungsstarker Nd:YAG-Laser (200 mJ - 420 mJ Pulsenergie), wird ein Laserstrahl mit einer Wellenlänge λ von 1064 nm erzeugt, der über eine Transfer- und Fokussierungsoptik **11** auf die Eisprobe **3** geleitet wird. Während des Laser-Beschusses wird Material aus der Oberfläche der zu untersuchenden Eisprobe 3 (der Fokus liegt optimal ca. 1 mm unter der Oberfläche) ablatiert und mit Hilfe eines inerten Trägergasstromes **12,** im Ausführungsbeispiel handelt es sich hierbei um das Edelgas Argon **Ar** (1,2 l/min Gasfluss), durch einen Kunststoffüberführungsschlauch **13** in das induktiv gekoppelte Plasma eines Massenspektrometers (z.B. **ICP-MS** System ELAN 6000 der Firma Perkin-EImer/Sciex, 1200 W - 1450 W Plasma-Leistung, Verweilzeit pro Masse 20 ms - 100 ms) überführt. Dort findet dann die massenspektrometrische Detektion von Spurenelementen im ablatierten Eisprobenmaterial statt.

Der Trägergasstrom **12** wird in einer Kühlbox **14** über einen Wärmetauscher **15** im angegebenen Ausführungsbeispiel auf eine Kühltemperatur **T**_{**k**} im Bereich von -30°C gekühlt. Eine in der Kühlbox **14** angefüllte Kühlflüssigkeit **16,** im gewählten Ausführungsbeispiel Ethanol C₂H₅OH oder Silikonöl, wird in einer Umlauf-Kühlvorrichtung **17,** beispielsweise Unistat 390 W der Firma Huber, wieder von der aufgenommenen Wärmelast befreit. Weiterhin durchfließt die Kühlflüssigkeit 16 noch die Probenkammer **2**, so dass auch hier eine sehr tiefe Temperatur herrscht. Durch beide Maßnahmen - Kühlung der Probenkammer **2** und des Trägergasstromes **12** - ist sicher gewährleistet, dass die gefrorene Eisprobe **3** während der Analyse nicht schmilzt oder antaut oder Probleme mit Spritzwasser auftreten.

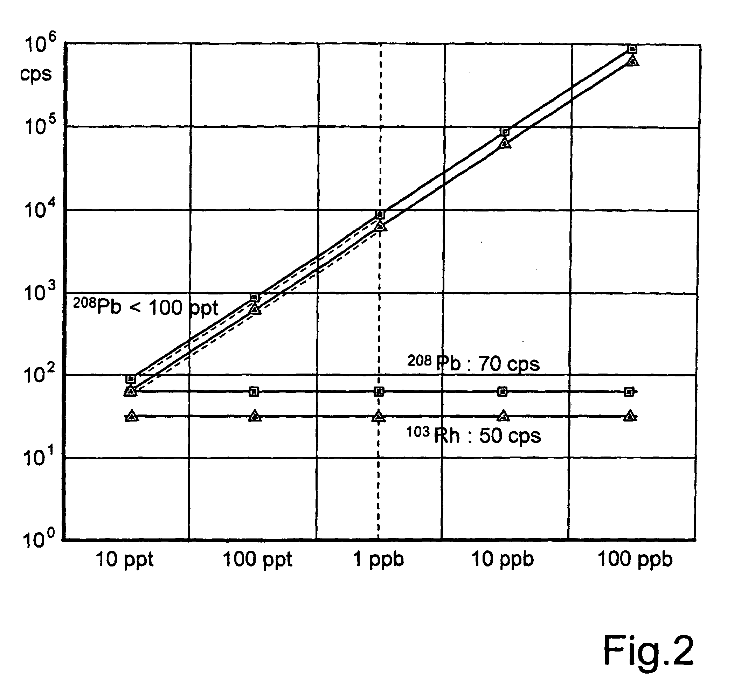

In der **Figur 2** ist ein Detektionsdiagramm für verschiedene Eisstandards zur Durchführung von Referenzmessungen dargestellt. In diesem Diagramm sind Zählraten in cps (counts per second) als Maß für die Intensität über der Elementkonzentration im Eisstandard in ppt (parts per trillion bzw. 1 ng/kg) bzw. ppb (parts per billion bzw. 1 µg/kg) aufgetragen. Erste Untersuchungen mit gefrorenen Standardlösungen zeigen, dass z.B. für eine Konzentration von 100 ppb eine Intensität von 800.000 cps für ²⁰⁸Pb erreicht werden kann, für ¹⁰³Rh 600.000 cps. Ein 10 ppb Eisstandard ergab im Mittel 80.000 cps für ²⁰⁸Pb; für ¹⁰³Rh 60.00 cps. Extrapoliert man die gefundenen Intensitäten unter der Annahme eines linearen Verlaufs, so kann mit den Bedingungen zum Zeitpunkt der Messwertaufnahme, die noch weiter optimierbar sind, beispielsweise für ²⁰⁸Pb eine Nachweisgrenze von unter 100 ppt aus der Festsubstanz gemessen werden. Der Untergrund auf diesen beiden Massen - als Maß für eine zu berücksichtigende Nullpunktverschiebung - beträgt für ²⁰⁸Pb 70 cps und für ¹⁰³Rh 50 cps. Die extrapolierten Intensitäten konnten experimentell verifiziert werden.

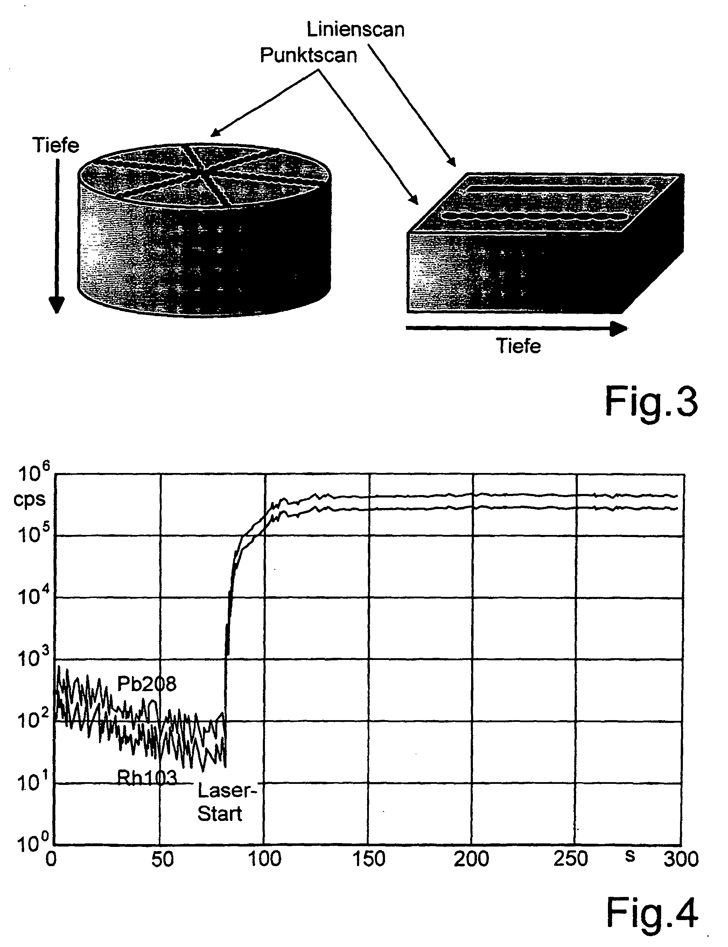

Der **Figur 3** sind verschiedene Ablationsmuster zu entnehmen. Beispielsweise kann auf einer Eisbohrkernscheibe (in der Figur links dargestellt) ein strahlenförmig angelegter Punktscan in einer Ebene durchgeführt werden. Die Messergebnisse geben dann z.B. Auskunft über die Kontamination des Bohrkernrandes durch den Bohrer. Bei einem Punktscan trifft der Laserstrahl auf einen definierten Punkt auf der Probenoberfläche und erzeugt mit der Zeit einen immer tiefer werdenden Krater. An einem Eiskemsegment (in der Figur rechts dargestellt) kann beispielsweise ein Punkt- oder ein Linienscan über die Tiefe durchgeführt werden. Bei einem Linienscan wird eine definierte Linie auf der Probenoberfläche immer wieder angefahren und gelasert.

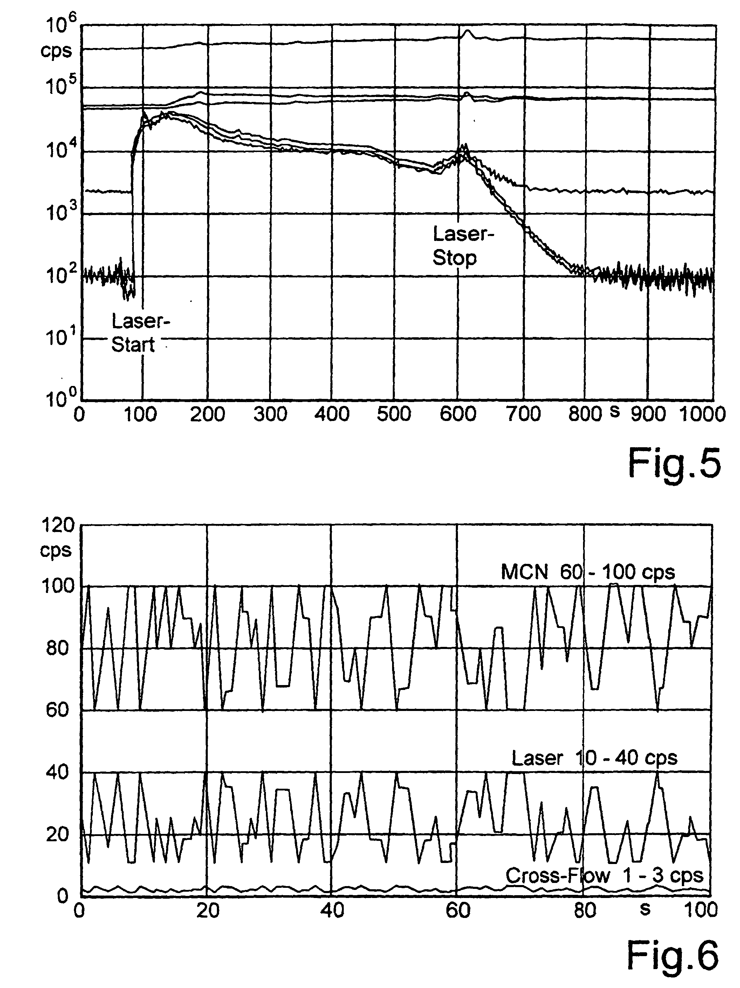

In der **Figur 4** sind die Detektionssignale (Intensität über der Zeit) für die Elemente Rhodium Rh und Blei Pb bei einem Linienscan eines 100 ppb Eisstandards dargestellt. Nach Einschalten des Lasers wird ein stabiler Signalverlauf bei einem Linienscan beobachtet. Die **Figur 5** verdeutlicht dagegen den Signalverlauf bei einem Punktscan für mehrere Elemente. Zu erkennen ist, dass mit zunehmender Tiefe beim Punktscan die Fokussierung des Laserstrahls nicht mehr stimmt und damit die Energiedichte auf der Probenoberfläche abnimmt. Als Folge davon wird weniger Material ablatiert und in das ICP transportiert, die Intensitäten gehen mit der Zeit zurück. Erste Untersuchungen von Eisproben ergaben eine hohe Signalstabilität für ¹⁷OH. Dieses Signal könnte entsprechend als Standardsignal eingesetzt werden.

In der **Figur 6** schließlich werden verschiedene Hintergrundspektren für unterschiedliche Probeneinführsysteme miteinander verglichen. Das durch Laserablation erzeugte Aerosol ist gewöhnlich trocken und führt bei dem in der Erfindung eingesetzten Einführsystem (Elan 6000) zu einem erhöhten Untergrund auf der Masse 220. Bei der Laserablation von Eis nimmt das Trägergas Argon beim Einleiten in die Probenkammer vermutlich Wasser aus der Probe auf. Ein Vergleich des Untergrundes verschiedener Probeneinführungssysteme zeigt, dass das durch Laserablation entstandene Aerosol einen Hintergrund von 10-40 cps erzeugt und damit unter den Werten eines mikrokonzentrischen Zerstäubers (MCN 6000, Firma CETAC) mit den Werten 60-100 cps, aber über denen eines Cross-Flow-Zerstäubers mit den minimalen Werten von 1-3 cps liegt. Der relativ niedrige Untergrund des Laser-Aerosols wirkt sich positiv auf die zu erreichenden Nachweisgrenzen aus. Für das Laser-Aerosol wurde eine Plasma-Leistung von 1450 W gewählt, analog der Einstellung für einen mikrokonzentrischen Zerstäuber.

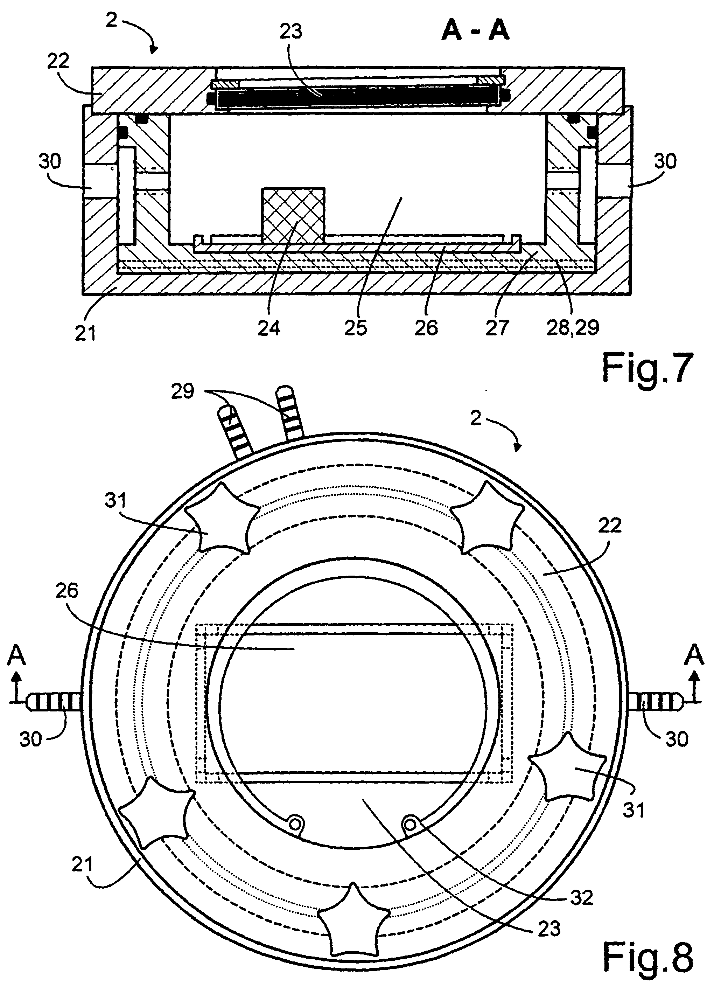

In der **Figur 7** ist die kühlbare Probenkammer **2** für eine bevorzugte Vorrichtung zur Durchführung des erfindungsgemäßen Laserablationsverfahrens im Querschnitt dargestellt. Sie besteht aus einem Probengehäuse **21** mit einem abnehmbaren Deckel **22**, die beide im gewählten Ausführungsbeispiel aus wärmeisolierendem, hochreinem Teflon hergestellt sind. Im Deckel **22** ist ein austauschbares Abdeckfenster **23** aus Quarz mittig angeordnet, durch das der Laserstrahl auf eine Eisprobe **24** gerichtet werden kann. Die Eisprobe **24** ist im eigentlichen Probenraum **25** auf einer Probenschale **26** positioniert, die ebenfalls aus hochreinem Teflon besteht. Da die Probenschale **26** einen sehr dünnen Boden aufweist, ist ihre Isolierwirkung gegenüber einem Metallblock **27,** auf dem die Probenschale **26** angeordnet, vernachlässigbar gering. Deshalb kann der Metallblock **27** gut als Kühlkörper, insbesondere aus gut wärmeleitendem, hochreinem Kupfer, ausgeführt sein und dazu ein integriertes Kanalsystem **28** aufweisen. Das Kühlmittel wird über Kühlanschlüsse **29** in das Kanalsystem **28** eingeleitet. Über Gasanschlüsse **30** wird das gekühlte Trägergas dem Probenraum **25** zu- und abgeführt. Die Eisprobe **24** kann also sowohl von unten als auch von oben sicher gekühlt werden.

Die **Figur 8** zeigt eine Draufsicht auf den Deckel **22** der Probenkammer **2** und deren Schnittebene **A-A** (ohne die Eisprobe **24).** Neben den Gasanschlüssen **30** sind die beiden Kühlanschlüsse **29** dargestellt. Über fünf Schnellverschlüsse **31** ist der Deckel **22** mit dem Probengehäuse **21** druckdicht verschraubbar. Zur Druckabdichtung sind diverse, einzeln nicht weiter bezeichnete Dichtringe vorgesehen. In der Mitte des Deckels **22** ist das austauschbare Abdeckfenster **23** zu erkennen, das über einen Sicherungsring **32** fixiert ist. Durch das Abdeckfenster **23** hindurch ist die Probenschale **26** zu erkennen.

Bei einer anderen, hier nicht dargestellten Ausführungsform trägt der Deckel der Probenkammer direkt ein Außengewinde. Der Deckel kann dann einfach und schnell in ein entsprechendes Innengewinde der Probenkammer ein- und ausgeschraubt werden, zusätzliche Schraubverbindungen und Verschlüsse entfallen. Das Schneiden von Gewinde direkt in das Teflonmaterial ist unproblematisch und sehr genau ausgeführt werden. Das Gewinde weist eine gute Führungsstabilität auf.

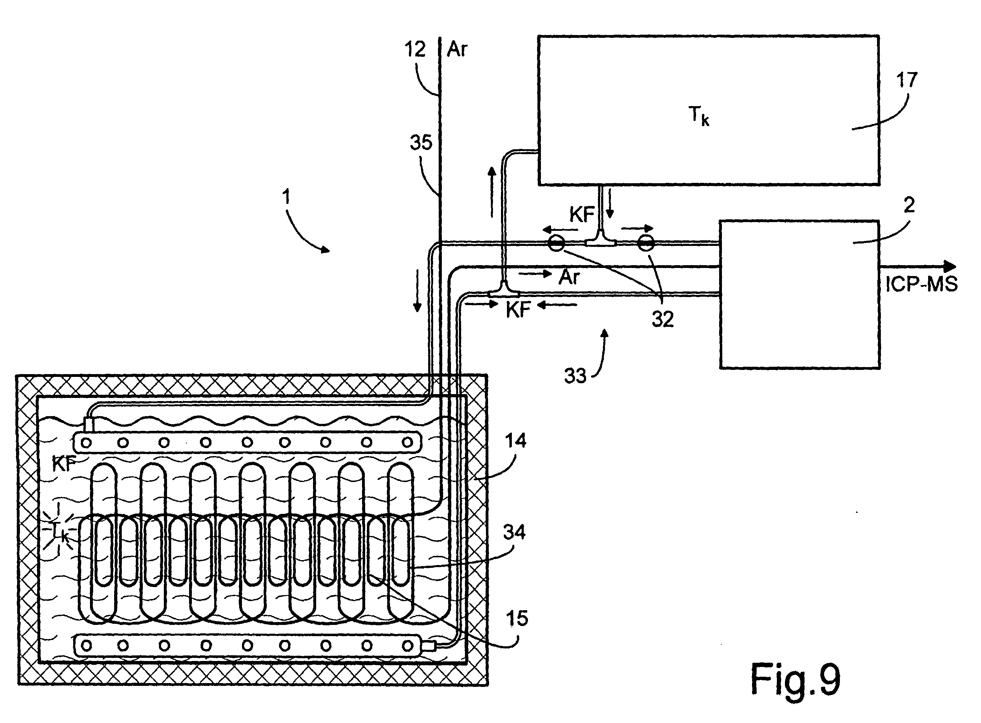

In der **Figur 9** ist schematisch das Kühlsystem der Vorrichtung **1** dargestellt (vergleiche Figur 1). Es besteht aus der Umlauf-Kühlvorrichtung **17,** die über zwei Ventile **32** mit einem Kühlmittel-Kreislauf **33** verbindbar ist. Als Kühlflüssigkeit **16** (KF) wird bevorzugt Ethanol oder Silikonöl verwendet, die Kühltemperatur **T**_{**k**} liegt bei -30°C. Alle kalten Rohrverbindungen sind wärmeisoliert, beispielsweise in der Ausführung als Amaflex-Schläuche. Von der Umlauf-Kühlvorrichtung **17** kann die Kühlflüssigkeit **KF** - je nach Ventilstellung - sowohl durch die Probenkammer **2** als auch durch die Kühlbox **14** geleitet werden. Die Kühlbox **14** ist mit Kühlflüssigkeit **KF** angefüllt und enthält den Wärmetauscher **15,** Dieser besteht im dargestellten Ausführungsbeispiel aus einer Kupfer-Kühlschlange **34,** die vom Trägergasstrom **12,** insbesondere Argon **Ar,** durchströmt wird. Das Argon **Ar** wird über eine Zuleitung **35** angeliefert, durchströmt nach seiner Abkühlung in der Kühlbox **14** die Probenkammer **2** und transportiert die ablatierten Probenteilchen zum **ICP-MS,** wo die massenspektrometrische Analyse stattfindet.

### Bezugszeichenliste

- 1: Vorrichtung
- 2: Probenkammer
- 3: Feststoffprobe
- 4: Steuerungsrechner
- 5: Laseranordnung
- 6: Justagelaser
- 7: Überwachungssystem
- 8: Farb-Kamera
- 9: Video-Monitor
- 10: Detektionslaser
- 11: Transfer- und Fokussierungsoptik
- 12: inerter Trägergasstrom
- 13: Kunststoffüberführungsschlauch
- 14: Kühlbox
- 15: Wärmetauscher
- 16: Kühlflüssigkeit
- 17: Umlauf-Kühlvorrichtung
- 21: Probengehäuse
- 22: Deckel
- 23: Abdeckfenster
- 24: Eisprobe
- 25: Probenraum
- 26: Probenschale
- 27: Metallblock
- 28: Kanalsystem
- 29: Kühlanschluss
- 30: Gasanschluss
- 31: Schnellverschluss
- 32: Ventil
- 33: Kühlmittel-Kreislauf
- 34: Kupfer-Kühlschlange
- 35: Zuleitung

- x,y,z: Raumrichtungen
- Ar: Argon
- ICP-MS: induktiv gekoppeltes Plasma- Massenspektrometer-System
- Tₖ: Kühltemperatur
- KF: Kühlflüssigkeit

## Patentansprüche

1. Analyseverfahren zur Detektion von räumlichen Spurenelement-Verteilungsmustern in einer Feststoffprobe (3) mittels rechnergestütztem, ortsaufgelöstem Abtragen von partikulärem Probenmaterial durch den Beschuss mit Laserstrahlung in einer von einem inerten Trägergasstrom (12) durchströmten Probenkammer (2) und anschließender Überführung des herausgelösten Probenmaterials mit Hilfe des Trägergasstromes (12) in ein induktiv gekoppeltes Plasma als Ionisierungsquelle zur Messdatenaufnahme durch ein Massenspektrometer (ICP-MS),
**dadurch gekennzeichnet, dass**
die Feststoffprobe (3) in Form einer natürlichen Eisprobe (24) oder einer gefrorenen biologischen Probe mit charakteristisch festem Ausgangszustand in der Probenkammer (2) angeordnet ist und zur Aufrechterhaltung des festen Ausgangszustandes der Feststoffprobe (3) während der Verfahrensdurchführung das Innere der Probenkammer (2) und der die Probenkammer (2) durchströmende Trägergasstrom (12) auf Temperaturen (T_{K}) unterhalb des Gefrier- oder Erstarrungspunktes der Feststoffprobe (3) gekühlt wird.

2. Analyseverfahren nach Anspruch 1,
**dadurch gekennzeichnet, dass**
die Kühltemperatur (T_{K}) in einem Temperaturbereich bis zu 30°C unterhalb des Gefrier- oder Erstarrungspunktes der Feststoffprobe (3) liegt.

3. Analyseverfahren nach Anspruch 1 oder 2,
**dadurch gekennzeichnet, dass**
die Kühlung durch eine geeignete Kühlflüssigkeit (KF,16), insbesondere Ethanol oden Silikonöl, erfolgt.

4. Analyseverfahren nach mindestens einem der Ansprüche 1 bis 3,
**dadurch gekennzeichnet, dass**
der Trägergasstrom (12) von dem Edelgas Argon (Ar) gebildet ist.

5. Analyseverfahren nach mindestens einem der Ansprüche 1 bis 4,
**dadurch gekennzeichnet, dass**
die Wellenlänge (λ) des emittierten Laserlichts im optimalen Absorptionsbereich der Feststoffprobe (3), insbesondere im infraroten Bereich, liegt.

6. Analyseverfahren nach mindestens einem der Ansprüche 1 bis 5,
**dadurch gekennzeichnet, dass**
Standardproben zur Durchführung von Referenzmessungen durch wiederholtes Aufsprühen einer fein zerstäubten Materiallösung auf einen Objektträger bei der gewählten Kühltemperatur (T_{K}) bis zur Erreichung einer vorbestimmten Schichtdicke oder durch Schockgefrieren einer ca. 1 cm hohen Materiallösung in einer Petrischale hergestellt werden.

7. Vorrichtung (1) zur Durchführung des Analyseverfahrens nach mindestens einem der vorangehenden Ansprüche 1 bis 6 zur Detektion von Spurenelementen in einer Feststoffprobe (3) mittels rechnergestützter, ortsaufgelöster Materialverdampfung durch Laserstrahlbeschuss mit einer justierbaren Laseranordnung (5, 6, 10) mit Transfer- und Fokussierungsoptik (11) durch ein Abdeckfenster (23) hindurch in eine Probenkammer (2), die auf einem im Raum (x,y,z) verfahrbaren Tisch angeordnet ist, und anschließender Überführung des verdampften Probenmaterials in einem Überführungssystem (13) mit Hilfe eines inerten Trägergasstromes (12, Ar) in ein induktiv gekoppeltes Plasma zur Messdatenaufnahme durch ein Massenspektrometer (ICP-MS) und mit einem Steuerungs- und Überwachungssystem (4, 8, 9),
**dadurch gekennzeichnet, dass**
die Probenkammer (2) aus einem wärmeisolierenden, hochreinen Material besteht und einen abnehmbaren Deckel (22) sowie eine im Innern positionierbare Probenschale (26) aufweist, unter der ein wärmeleitender, hochreiner Metallblock (27) mit einem integrierten, Anschlüsse (29) aufweisenden Kanalsystem (28) angeordnet ist, und dass eine über Ventile (32) zuschaltbare Umlauf-Kühlvorrichtung (17) mit einer Kühlflüssigkeit (16, KF) vorgesehen ist, die über wärmeisolierende Rohrverbindungen sowohl mit dem Kanalsystem (28) in dem Metallblock (27) als auch mit einer externen Kühlbox (14) verbunden ist, die in ihrem Innern einen Wärmetauscher (15) aufweist, der auf seiner wärmeren Seite mit der Trägergaszuleitung (35) verbunden ist.

8. Vorrichtung zur Durchführung des Analyseverfahrens nach Anspruch 7,
**dadurch gekennzeichnet, dass**
als wärmeisolierendes, hochreines Material für die Probenkammer (2, 21, 22) und die Probenschale (26) Teflon und als wärmeleitendes, hochreines Material für den Metallblock (27) Kupfer ausgewählt ist.

9. Vorrichtung zur Durchführung des Analyseverfahrens nach Anspruch 7 oder 8
**dadurch gekennzeichnet, dass**
der Laser (10) der Laseranordnung (5) als Infrarot-Laser, insbesondere auch in modifizierter Form, ausgebildet ist.

10. Vorrichtung zur Durchführung des Analyseverfahrens nach mindestens einem der vorangehenden Ansprüche 7 bis 9,
**dadurch gekennzeichnet, dass**
als Teil der Laseranordnung (5) ein Justagelaser (6) vorgesehen ist, der Laserlicht im sichtbaren Wellenlängenbereich emittiert.

## Claims

1. Analytical method of detecting spatial trace element distribution patterns in a solid matter sample (3) by computer-assisted spatially resolved ablation of particulate sample material by laser beam bombardment in a sample chamber (2) permeated by a stream (12) of inert carrier gas and subsequent transfer of the separated sample material by the carrier gas stream (12) into an inductively coupled plasma as an ionization source for recording measurements by a mass spectrometer (ICP-MS),
**characterized by the fact that**
the solid matter sample (3) formed as a natural ice sample (24) or as a frozen biological sample of a characteristic solid initial state is disposed in a sample chamber (2) and that for maintaining the solid initial state of the solid matter sample (3) during execution of the method the interior of the sample chamber (2) and the stream (12) of carrier gas permeating the sample chamber (2) are cooled to temperatures (Tₖ) below the melting or solidification point of the solid matter sample (3).

2. Analytical method according to claim 1,
**characterized by the fact that**
the cooling temperature (Tₖ) lies in a temperature range up to 30□C below the melting or solidification point of the solid matter sample (3).

3. Analytical method according to claim 1 or 2,
**characterized by the fact that**
that cooling is carried out by a suitable cooling liquid (KF, 16), especially ethanol or silicon oil.

4. Analytical method in accordance with at least one of claims 1 to 3,
**characterized by the fact that**
the stream (12) of carrier gas is constituted by the noble gas argon (Ar).

5. Analytical method in accordance with at least one of claims 1 to 4,
**characterized by the fact that**
the wavelength (λ) of the emitted laser light lies within the optimum absorption range of the solid matter sample (3), especially in the infrared range.

6. Analytical method in accordance with at least one of claims 1 to 5,
**characterized by the fact that**
standard samples are produced for carrying out reference measurements by repeated spraying of a finely dispersed material solution on an object support at the selected cooling temperature (Tₖ) until establishment of a predetermined layer thickness or by flash cooling of a material solution of a height of about 1 cm in a Petri dish.

7. Apparatus (1) for practicing the analytical method in accordance with at least one of the preceding claims 1 to 6 for the detection of trace elements in a solid matter sample (3) by a computer-assisted spatially resolved material nebulization by laser beam bombardment with an adjustable laser arrangement (5, 6, 10) with transfer and focussing optics (11) through a cover window (23) into a sample chamber (2) disposed on a three-dimensionally (x, y, z) movable table, and subsequent transfer of the nebulized sample material in a transfer system (13) by a stream (12) of an inert carrier gas (Ar) into an inductively coupled plasma for measurement data recording by a mass spectrometer (ICP-MS) and with a control and monitoring system (4, 8, 9)
**characterized by the fact that** the sample chamber (2) consists of a heat-insulating super pure material and is provided with a removable lid (22) as well as with a sample dish (26) positionable in the interior thereof below which there is disposed a heat-conductive super pure metal block (27) with an integrated channel system (28) provided with connections (29) and that there is provided a recirculating cooling device (17) with a cooling liquid (16, KF) connectable by valves (32) and connected by heat-insulating pipe connections to the channel system (28) in the metal block (27) as well as to an external cooling box (14) provided in its interior with a heat exchanger (15) connected at its warmer side to the feed line (35) of the carrier gas.

8. Apparatus for practicing the analytical method according to claim 7,
**characterized by the fact that**
Teflon® is selected as the heat-insulating super pure material for the sample chamber (2, 21, 22) and copper is selected as the heat-conductive super pure material for the metal block (27).

9. Apparatus for practicing the analytical method according to claim 7 or 8,
**characterized by the fact that**
the laser (10) of the laser arrangement (5) is structured as an infrared laser, especially in modified form.

10. Apparatus for practicing the analytical method according to at least one of the preceding claims 7 to 9,
**characterized by the fact that**
as part of the laser arrangement (5) there is provided an adjustable laser (6) which emits laser light in the range of visible wavelengths.

## Revendications

1. Procédé d'analyse permettant la détection de motifs de répartition spatiale d'oligo-éléments dans un échantillon solide (3), par enlèvement assisté par ordinateur, d'emplacement résolu, d'un matériau particulaire d'échantillon par bombardement par faisceau laser dans une chambre à échantillons (2) traversée par un gaz porteur (12), et par transfert successif du matériau échantillon à l'aide du flux de gaz porteur (12) dans un plasma couplé par induction en tant que source d'ionisation, pour la prise de données de mesure par un spectromètre de masse (ICP-MS),
**caractérisé en ce que**
l'échantillon solide (3) est disposé dans la chambre à échantillons (2) sous la forme d'un échantillon naturel de glace (24) ou un échantillon biologique congelé d'état initial fixe de manière caractéristique, et pour conserver l'état initial fixe de l'échantillon solide (3) pendant l'exécution du procédé, l'intérieur de la chambre à échantillons (2) et le flux de gaz porteur (12) traversant la chambre à échantillons (2) sont refroidis à des températures (T_{K}) inférieures au point de congélation ou de solidification de l'échantillon solide (3).

2. Procédé d'analyse selon la revendication 1,
**caractérisé en ce que**
la température de refroidissement (T_{K}) figure dans une plage de températures s'étendant jusqu'à 30 °C en dessous du point de congélation ou de solidification de l'échantillon solide (3).

3. Procédé d'analyse selon la revendication 1 ou 2,
**caractérisé en ce que**
le refroidissement se réalise par un liquide de refroidissement adapté (KF, 16), en particulier de l'éthanol ou de l'huile de silicone.

4. Procédé d'analyse selon l'une au moins des revendications 1 à 3,
**caractérisé en ce que**
le flux de gaz porteur (12) est constitué du gaz rare Argon (Ar).

5. Procédé d'analyse selon l'une au moins des revendications 1 à 4,
**caractérisé en ce que**
la longueur d'onde (λ) de la lumière laser émise figure dans la plage d'absorption optimale de l'échantillon solide (3), en particulier dans la plage des infrarouges.

6. Procédé d'analyse selon l'une au moins des revendications 1 à 5,
**caractérisé en ce que**
les échantillons standard pour effectuer les mesures de référence sont fabriqués par pulvérisation répétée sur un porte-objet à la température choisie (T_{K}) d'une solution de matériau finement pulvérisée jusqu'à l'obtention d'une épaisseur de couche prédéterminée ou par congélation ultrarapide d'une solution de matériau d'une hauteur d'environ 1 cm dans une boîte de Pétri.

7. Dispositif (1) servant à mettre en oeuvre le procédé d'analyse selon l'une au moins des revendications précédentes 1 à 6 pour détecter des oligo-éléments dans un échantillon solide (3) par évaporation de matériau assisté par ordinateur, d'emplacement résolu, par bombardement par faisceau laser avec un dispositif laser réglable (5, 6, 10) comportant une optique de transfert et de focalisation (11) à travers une fenêtre de recouvrement (23) dans une chambre à échantillons (2), disposée sur une table pouvant être déplacée dans l'espace (x, y, z), et transfert successif du matériau d'échantillons évaporé dans un système de transfert (13) à l'aide d'un flux de gaz rare porteur (12, Ar) dans un plasma couplé par induction pour la prise de données de mesure par un spectromètre de masse (ICP-MS), et comportant un système de commande et de surveillance (4, 8, 9),
**caractérisé en ce que**
la chambre à échantillons (2) se constitue d'un matériau isolant thermique à haut degré de pureté et d'un couvercle amovible (22) ainsi que d'une boîte à échantillons (26) pouvant être positionnée à l'intérieur, sous laquelle est disposé un bloc métallique (27) à haut degré de pureté, conduisant la chaleur, avec un système de canal (28) présentant des raccordements intégrés (29), et un dispositif de refroidissement à circulation (17) avec un liquide de refroidissement (16, KF) peut être branché raccordé par des soupapes (32), et relié par des liaisons par tuyau isolantes thermiquement tant au système de canal (28) dans le bloc métallique (27) qu'également à une boîte externe de refroidissement (14) dont l'intérieur contient un échangeur de chaleur (15) est relié en son côté le plus chaud à une conduite de gaz porteur (35).

8. Dispositif servant à mettre en oeuvre le procédé d'analyse selon la revendication 7,
**caractérisé en ce que**
comme matériau isolant thermique à haut degré de pureté on choisit du Téflon pour les chambres à échantillons (2, 21, 22) et les boîtes à échantillons (26), et du cuivre comme matériau conduisant la chaleur, à haut degré de pureté, pour le bloc métallique (27).

9. Dispositif servant à mettre en oeuvre le procédé d'analyse selon la revendication 7,
**caractérisé en ce que**
le laser (10) du dispositif laser (5) est un laser infrarouge, en particulier également de forme modifiée.

10. Dispositif servant à mettre en oeuvre le procédé d'analyse selon l'une au moins des revendications précédentes 7 à 9,
**caractérisé en ce que**
comme partie du dispositif laser (5), émet un laser de réglage (6) émet de la lumière laser dans la plage des longueurs d'onde visibles.
